# FASCICULE DE BREVET EUROPEEN

(11) **EP 0 664 112 B1**
(45) Date de publication et mention de la délivrance du brevet: **26.03.1997**
(21) Numéro de dépôt: 94402811.7
(22) Date de dépôt: 07.12.1994
(51) Int. Cl.: A61K 7/00, A61K 7/48

(54) **Poudre cosmétique ou dermatologique, son procédé de préparation et utilisations**
Kosmetisches oder dermatologisches Pulver, Verfahren zu seiner Herstellung und Verwendungen
Cosmetic and dermatological powder, its process for manufacturing and its use

(30) Priorité: 22.12.1993 EP 93121169
(43) Date de publication de la demande: 26.07.1995
(73) Titulaire: L'OREAL, 75008 Paris (FR)
(72) Inventeur: Masson, Gérard, 1096 Cully (CH); Candau, Didier, 91570 Bievres (FR); Khayat, Carine, 94210 La Varenne (FR)
(74) Mandataire: Lhoste, Catherine

(56) Documents cités:
- EP-A- 0 315 541
- EP-A- 0 472 225
- CH-A- 663 725
- FR-A- 2 068 447
- FR-A- 2 112 759
- FR-A- 2 649 318
- FR-A- 2 675 044
- PATENT ABSTRACTS OF JAPAN vol. 10 no. 355 (C-388) & JP-A-61 155307 (LION CORP.) 15 Juillet 1986,
- DATABASE WPI Week 1089 Derwent Publications Ltd., London, GB; AN 89-071649 & JP-A-01 022 814 (KAMEYAMINAMI CHAIN) , 25 Janvier 1987

## Description

L'invention se rapporte à une poudre cosmétique ou dermatologique et à un procédé de préparation de cette poudre. Celle-ci peut être utilisée pour l'obtention de capsules notamment ingérables. L'invention concerne plus spécialement une émulsion huile-dans-eau (H/E) déshydratée, utilisable telle que et susceptible d'être reconstituée.

Les produits anhydres communément utilisés en cosmétique sont huileux (eau-dans-huile) et présentent un toucher gras. Ces produits sont assez bien adaptés à une application sur peaux sèches.

Malheureusement ils ne sont ni sympathiques ni pratiques à manipuler car sont souvent fluides voir liquides. Par ailleurs, ces produits ne sont pas satisfaisants pour l'hydratation des peaux normales ou grasses, ils pénètrent mal dans la peau et laissent un film gras et brillant.

Le but que vise à résoudre l'invention est une poudre cosmétique ou dermatologique anhydre, qui bénéficie des qualités notamment restructurantes de tels produits, qui peut contenir au moins un corps gras sans avoir les inconvénients tel que la «lourdeur», une fois appliquée sur la peau, des produits anhydres de l'art antérieur.

Un objet de l'invention est donc l'utilisation d'un agent servant à la fois d'agent structurant et émulsionnant choisi parmi les biopolymères, dans une poudre cosmétique ou dermatologique contenant outre cet agent, au moins une matière grasse, au moins une substance cosmétiquement ou dermatologiquement active, la dite poudre contenant au moins 1% en poids de matière grasse et de substances actives, la dite poudre étant susceptible d'être obtenue à partir d'une émulsion huile-dans-eau homogénéisée et déshydratée, et ladite poudre étant susceptible de reconstituer par hydratation ladite émulsion huile-dans-eau.

Un autre objet de l'invention est une poudre cosmétique ou dermatologique obtenue à partir d'une émulsion huile-dans-eau homogénéisée et déshydratée, la dite poudre contenant au moins un biopolymère servant à la fois d'agent structurant et d'agent émulsionnant, choisi parmi les protéines d'origine animale ou végétale et leurs dérivés ou hydrolysats, au moins un polysaccharide à l'exception des cyclodextrines, servant à la fois d'agent structurant et d'agent émulsionnant, au moins une matière grasse, au moins une substance cosmétiquement ou dermatologiquement active, la dite poudre contenant au moins 1% en poids de matière grasse et de substances actives et étant susceptible de reconstituer par hydratation ladite émulsion huile-dans-eau.

Par émulsion, on entend aussi toute dispersion d'huile dans eau.

Certes, il est connu par les documents FR-A-2068447 et FR-A-2649318 de préparer des poudres à partir d'émulsions huile-dans-eau, susceptibles de redonner les dites émulsions par hydratation.

Par ailleurs, le document FR-A-2675044 décrit des poudres obtenues par trituration à sec et non à partir d'une émulsion. Le document JP-A-61-155307 décrit une poudre contenant de l'huile absorbée sur de la silice et le document JP-A-01-22814 décrit la préparation d'une poudre d'huile essentielle, facilement soluble dans l'eau, par mélange de l'huile essentielle avec de l'eau et une protéine puis séchage de la composition obtenue.

Enfin, le document EP-A-315541 décrit l'obtention de poudre de yaourt susceptible d'être réhydratée au moment de l'emploi pour donner une composition cosmétique.

La poudre selon l'invention présente notamment les avantages d'être applicable sur des peaux grasses ou normales, d'être facile et rapide à utiliser, aisément dosable, de conserver les propriétés hydratantes des corps gras et de ne pas nécessiter l'ajout obligatoire d'un liquide tel que l'eau ; elle peut être utilisée telle qu'elle.

Une telle présentation d'un produit cosmétique permet l'incorporation de composés ayant différentes propriétés physico-chimiques.

En effet, de telles poudres peuvent contenir simultanément des corps gras et des détergents. Ceci permet notamment de nettoyer la peau tout en l'hydratant et la restructurant. On a donc à faire à un nouveau concept de produit cosmétique ou dermatologique d'applications variées, non spécifique à un type de peau.

Par ailleurs, ces poudres permettent des conservations stables, de longue durée, de produits instables en milieu aqueux (vitamines par exemple) et ainsi d'éviter la détérioration de leur activité. Dans l'art antérieur, ces produits sont isolés de l'eau (conditionnement séparé par exemple).

La poudre selon l'invention présente en outre l'avantage d'avoir un volume faible notamment après compactage, ce qui en facilite la manipulation.

La poudre selon l'invention peut éventuellement être mélangée avec un liquide à base d'eau. Par liquide à base d'eau, on entend un liquide choisi parmi l'eau, le lait, les jus de légumes ou de fruits et les décoctions et infusions de plantes. En général, la préparation de l'émulsion, donc de sa phase aqueuse, et son hydratation sont réalisées avec de l'eau.

L'émulsion qui aura été déshydratée doit avoir été traitée de manière particulière, de façon à ce que la matière grasse et la substance cosmétiquement active soient encapsulées dans une matrice. Par substance encapsulée, on entend tout liquide ou tout solide susceptible d'être retenu entier ou finement dispersé à l'intérieur d'une structure solide tenant lieu de charpente, de support ou d'enveloppe dans un but de protection ou de conservation.

La poudre selon l'invention peut avoir une humidité résiduelle qui se situe entre 2 et 4 %.

La poudre selon l'invention contient une substance cosmétiquement active. Elle peut être soit la phase lipidique en tant que telle comme les huiles essentielles, soit un agent cosmétique au sens propre du terme, soit une combinaison des deux. La poudre de l'invention peut contenir entre 1 et 94 % et de préférence entre 10 et 70 % de matière grasse et de substances cosmétiquement actives. Tous les pourcentages mentionnés dans la présente invention sont des pourcentages en poids.

La matière grasse utilisée peut être choisie parmi les huiles minérales telles que les huiles de paraffine ou la vaseline, les huiles de silicones, les huiles végétales telles que l'huile de coco, d'amandes douces, d'abricot, de maïs, de jojoba, d'olive, d'avocat, de sésame, de palme, d'eucalyptus, de romarin, de lavande, de pin, de thym, de menthe, de cardamone, de fleur d'oranger de soja, de son, de riz, de colza et de ricin, les huiles ou graisses animales telles que le suif, la lanoline, l'huile de beurre, les esters d'acides gras, les esters d'alcool gras, les cires dont le point de fusion est celui de la peau (les cires animales comme la cire d'abeille, les cires de carnauba ou de candellila, les cires minérales comme les cires micro-cristallines et les cires de synthèse comme les cires de polyéthylène ou de silicone). On peut aussi utiliser toutes les huiles acceptables en cosmétique, par exemple celles mentionnées dans l'ouvrage CTFA, Cosmetic Ingredient Handbook, 1ère édition, 1988, The Cosmetic, Toiletry and Fragrance Association, Inc., Washington.

Par substance cosmétiquement ou dermatologiquement active présente dans l'émulsion au sens propre du terme, on entend les actifs cosmétiques choisis parmi les agents anti-acné, anti-microbien, anti-transpiration, astringents, déodorants, dépilatoires, analgésiques externes, les agents de conditionnement des cheveux, de conditionnement de la peau, de protection solaire, les vitamines, les catéchines, les flavonoïdes, les céramides, les matières grasses, les acides gras polyinsaturés, les acides gras essentiels, les agents kératolytiques, les enzymes, les anti-enzymes, les hydratants, les anti-inflammatoires, les détergents ou agents moussants, les parfums, les charges matifiantes minérales ou synthétiques. Ces substances représentent notamment de 1 à 20 % de la poudre.

Les agents détergents ou moussants sont notamment le sel disodique de cocoamphodiacétate (MIRANOL C2M de RHONE POULENC) ; le sel disodique du lauroyléther sulfosuccinate (SETACIN 103 de ZSCHIMMER) ; les acylates de protéines végétales, notamment les acylates de protéines de soja (PROTEOL VS22 de SEPPIC) ; le sel de triéthanolamine du cocoyl glutamate (acylglutamate CT12 d'AJINOMOTO) ; le sel de sodium du lauroyl sarcosinate (ORAMIX L30 de SEPPIC) ; le décyl-éther de glucoside (ORAMIX NS10 de SEPPIC) ; le lauroyl éther sulfate de sodium (NEOPON LOS RO de WITCO).

On peut aussi utiliser des composés actifs pâteux comme les dérivés de lanoline (lanoline acétylée), la lanoline et les alcools de lanoline ; les dérivés du cholestérol notamment les esters de cholestérol (12 hydroxy stéarate de cholestéryl) ; les composés pâteux d'origine synthétique : les esters hydroxylés du pentaérythritol (SALACOS 168M), les mono-esters linéaires comme le stéarate de butyle, le propionate d'arachidyle ou l'heptanoate de stéaryle, les triglycérides à chaîne grasse inférieure à C₁₆.

Ces substances sont soit hydrosolubles, soit liposolubles, soit à la fois liposolubles et hydrosolubles soit dispersibles. Elles peuvent être notamment choisies parmi les composés mentionnés dans l'ouvrage précité CTFA aux pages 51 à 101.

L'agent structurant et émulsionnant présent dans la poudre selon l'invention peut être présent en une quantité allant de 4 à 30 % et de préférence en une quantité allant de 4 à 15 % en poids.

Ces composés qui possèdent à la fois les deux propriétés peuvent être choisis parmi les hydrocolloïdes et les biopolymères. Par hydrocolloïdes, on entend des polysaccharides qui peuvent être modifiés notamment par au moins une chaîne hydrophobe.

Par biopolymères, on entend essentiellement les protéines d'origine animale ou végétale et leurs dérivés ou hydrolysats, comme le caséinate de sodium, les protéines de soja, de blé.

La poudre peut contenir un agent de surface mouillant destiné à favoriser la mouillabilité de la poudre lors de la reconstitution de l'émulsion. Cet agent est normalement présent en une quantité allant de 0 à 30 % en poids et mieux de 1 à 20 %. Cet agent de surface permet entre autre, d'augmenter la vitesse de réhydratation de la poudre servant de la dispersion ou émulsion.

Cet agent de surface peut être choisi parmi les agents de surface hydrophiles comme les glycols tels que l'héxylène glycol, le butylène glycol-1,2, le sulfosuccinate d'éthyl-2-hexyle ; octylphénol oxyéthyléné (9), les sels des dérivés cocoyle ou lauroyle collagénique, le palmitate de sorbitan et les dérivés polyoxyéthylénés des esters de sorbitan, les sels d'ammonium quaternaire à chaîne grasse.

La poudre selon l'invention peut aussi comprendre d'autres excipients tels que des colorants, des abrasifs, des opacifiants, des complexants.

Un autre objet de l'invention concerne un procédé de préparation de la poudre telle que définie précédemment.

Ce procédé consiste à former une émulsion huile-dans-eau, à homogénéiser cette émulsion, à déshydrater ladite émulsion pour former ladite poudre.

Selon un mode de réalisation du procédé, l'émulsion utilisée est telle qu'elle a une teneur en matière sèche comprise entre 5 et 70 % en poids et de préférence comprise entre 10 et 60 % en poids. Une telle teneur en matière sèche permet d'avoir une viscosité de l'émulsion telle qu'elle soit suffisamment fluide pour pouvoir être pompée.

Cette teneur en matière sèche donne, dans l'émulsion, une teneur en matière grasse ou en substance cosmétiquement ou dermatologiquement active comprise entre 0,1 et 51 % en poids, et une teneur en agent structurant et émulsionnant comprise entre 0,1 et 18% en poids.

La phase aqueuse peut être préparée à toute température (0 à 85° C) du moment qu'à la température utilisée, l'agent structurant et émulsionnant soit soluble. On peut travailler à une température comprise entre 40 et 80° C, de manière à avoir une dissolution plus rapide de certains ingrédients.

Il est également possible de préparer le mélange à une température plus basse, d'environ 4 à 10°C, afin de maintenir de bonnes conditions de sécurité bactériologiques.

De manière parallèle, on prépare une phase grasse à la même température que la phase aqueuse, qu'on ajoute à la phase aqueuse en une quantité telle qu'on a, dans l'émulsion qui en résulte, une teneur en matière sèche comprise entre 10 et 60 % en poids.

Ainsi la proportion des constituants telle que définie selon la présente invention garantit une bonne homogénéité et stabilité de l'émulsion, et permet également une excellente reconstitution de l'émulsion.

On forme alors une émulsion en mélangeant lentement les phases aqueuse et grasse, par exemple en incorporant doucement la phase grasse dans la phase aqueuse tout en maintenant une agitation constante et en l'absence de conservateur, une température d'environ 4 à 10° C et à 65 à 75° C environ, toujours pour des raisons bactériologiques.

On obtient ainsi une émulsion huile-dans-eau, présentant un pH dépendant de sa composition, mais généralement compris entre 4 et 9.

Le taux de matière sèche de ladite émulsion est de préférence compris entre 10 et 60 % de manière à permettre le pompage, l'homogénéisation et la déshydratation (ou séchage).

On homogénéise alors l'émulsion, sous pression élevée, de manière à diminuer la taille moyenne des gouttelettes de la phase grasse jusqu'à environ 1 µm, voire moins. On a observé que l'homogénéisation permet d'améliorer les qualités de réhydratation de la poudre déshydratée, ainsi que ses propriétés fonctionnelles comme, par exemple, l'aptitude à former un gel ferme et stable, ce qui est important dans la cas où l'on souhaite reconstituer un produit ayant l'apparence, la texture et la consistance d'un produit cosmétique d'aspect classique.

On peut employer un homogénéisateur à deux étapes, selon les méthodes habituelles. On peut toutefois prendre la précaution de ne pas dépasser une pression trop élevée, de l'ordre de 350 bars (35 X 10⁶ Pa), de manière à éviter une dénaturation de certains agents structurants et émulsionnants qui entraînerait une perte de fonctionnalité et conduirait à une séparation de phases irréversible de l'émulsion. L'homogénéisation peut être effectuée à une température située entre 4 et 45° C, mais préférentiellement entre 4 et 10° C pour des raisons de sécurité bactériologiques.

L'émulsion ainsi homogénéisée est alors déshydratée par tout procédé connu, tel que la lyophilisation ou l'atomisation, ou tout autre procédé de séchage sous vide. On obtient une poudre qui peut être éventuellement agglomérée par tout procédé connu. Les températures de l'air chaud utilisé pour le séchage sont de l'ordre de 100 à 160° C et la température de sortie de la poudre est de 30 à 90° C.

La poudre obtenue peut être également compactée pour gagner du volume et pour en faciliter le conditionnement, la conservation, le stockage et l'emploi. Avant le compactage éventuel de la poudre, celle-ci peut subir une étape de granulation supplémentaire ayant pour but d'homogénéiser la granulométrie de la poudre.

On obtient ainsi une poudre pouvant être utilisée directement ou, après reconstitution de l'émulsion par addition de liquide.

L'utilisation ultérieure de la poudre dépend des substances actives et éventuellement de la quantité de liquide qu'on ajoute, pour reconstituer l'émulsion, et donc de sa viscosité, qui en détermine la consistance, plus ferme pour une crème de soin ou une crème hydratante et plus ou moins liquide pour un lait démaquillant, une huile pour le visage ou un shampoing.

D'une manière générale, par addition de liquide dans un rapport poudre/liquide compris entre 10:1 et 1:1, on peut reconstituer un produit ayant la consistance d'un produit cosmétique ou dermatologique classique.

La poudre selon l'invention présente aussi l'avantage d'être immédiatement réhydratable à froid, dans un liquide tel que l'eau, et de permettre la reconstitution d'une émulsion homogène et stable, sans séparation de phase.

De plus, la poudre obtenue est très stable et peut se conserver pendant 2 à 8 semaines à température ambiante sans que l'on puisse observer de séparation de phase ou d'autres dégradations, microbiologiques par exemple. Il est possible de lui ajouter, après reconstitution, d'autres ingrédients cosmétiques ou dermatologiques.

Un dernier objet de l'invention est une utilisation de la poudre telle que définie précédemment dans des émulsions notamment cosmétiques ou dermatologiques ou dans des capsules notamment ingérables.

L'invention est illustrée plus en détail à l'aide des exemples qui suivent.

### Exemple 1:

On prépare un mélange contenant 60 g de caséinate de sodium, 30 g de gomme de xanthane et 695 g d'eau, que l'on mélange pendant 30 min à température ambiante. On ajoute à cette phase aqueuse 215 g d'huile d'abricot préalablement refroidie à 10 °C, tout en maintenant une agitation constante de manière à obtenir une émulsion huile dans-eau, présentant un pH de l'ordre de 7.

On ajoute un parfum et un colorant comme le β-carotène, on homogénéise et on déshydrate l'émulsion par vaporisation. On reconstitue éventuellement l'émulsion par addition d'une partie d'eau à une partie de poudre. On obtient une base de crème de soin. La base ainsi préparée reste stable, sans dégradation de l'actif, pendant au moins 8 heures.

### Exemple 2:

On opère comme dans l'exemple 1 avec la même phase aqueuse, mais on ajoute à cette phase 210 g d'huile d'abricots, 5 g de vitamine E. On homogénéise l'émulsion et on déshydrate comme précédemment. On obtient une émulsion sèche que l'on réhydrate à raison de 2 parties de poudre pour une partie d'eau pour obtenir une crème protectrice.

### Exemple 3:

On prépare une phase aqueuse contenant 60 g de caséinate de sodium, 5 g de carboxyméthylcellulose, 5 g de gomme de guar, 700 g d'eau et 15 g de glycérine que l'on mélange pendant 30 min à température ambiante. On ajoute à cette phase aqueuse une phase lipidique contenant 100 g d'huile d'abricot, 50 g d'huile de silicone et 65 g d'huile de paraffine, tout en maintenant une agitation constante de manière à obtenir une émulsion huile-dans-eau, présentant un pH de l'ordre de 7.

On homogénéise comme dans l'exemple 1 pour obtenir une émulsion ayant un taux de matière sèche de 28,5 %. On déshydrate l'émulsion par lyophilisation. On reconstitue le produit par addition d'une partie d'eau par partie de poudre et on obtient une crème nutritive parfaitement stable.

### Exemple 4:

On prépare une phase aqueuse contenant 60 g de caséinate de sodium, 50 g d'amidon de maïs et 590 g d'eau. On mélange cette phase aqueuse avec 300 g d'huile d'abricot qu'on homogénéise comme dans l'exemple 1.

On déshydrate par séchage par pulvérisation et on obtient une poudre contenant 2,5 % d'humidité résiduelle. On reconstitue l'émulsion par addition d'une partie de poudre pour 0,5 partie d'eau et on obtient un masque pour le visage. Dans l'émulsion reconstituée on peut ajouter selon l'art antérieur tout actif dermatologique ou cosmétique.

### Exemple 5:

On prépare l'émulsion de l'exemple 2 dans laquelle la phase grasse contient 175 g d'huile d'abricot, 5 g de vitamine E, 10 g de vitamine C et 15 g de β-carotène que l'on déshydrate par atomisation à une température d'entrée supérieure à 100° C et inférieure à 120° C. La poudre obtenue est encapsulée dans des capsules molles de gélatine ingérables, servant de compléments alimentaires vitaminés.

### Exemple 6:

| | |
|---|---|
| Triglycéride de vitamine F | 4,4 % |
| Conservateur | 0,5 % |
| Caséinate de sodium | 5,4 % |
| Eau | qsp 100 % |
| Gomme de xanthane et de caséine | 3,1 % |
| Gluconate de cuivre | 0,0005 % |
| Tyrosine | 0,02 % |
| Phénylalanine | 0,008 % |
| Tocophérol | 0,002 % |
| Niacinamide | 0,002 % |
| Acide ascorbique | 0,06 % |
| β-carotène | 0,002 % |

Après formation de l'émulsion H/E de façon classique, celle-ci est homogénéisée et séchée par atomisation ; la poudre obtenue peut être mise sous forme de comprimés par tous les moyens connus.

La poudre obtenue peut également être encapsulée dans une capsule molle de gélatine.

Les deux formes de présentation servent de compléments vitaminés pour la cosmétique orale.

### Exemple 7 :

| | |
|---|---|
| Huile minérale | 8 % |
| Conservateurs | 0,5 % |
| Caséinate de sodium | 5,4 % |
| Eau | qsp 100,00 % |
| Gomme de xanthane et de caséine | 3,1 % |
| Lauryl éther sulfate de sodium à 2,2 moles d'oxyde d'éthylène | 5,0 % |
| Dodécanediol polyglycérolé à 3,5 moles de glycérol | 6,3 % |
| Acide Ethylènediaminetétracétique | 0,1 % |
| Hydroxyéthyl cellulose réticulée et quaternisée | 0,2 % |

Après homogénéisation, l'émulsion H/E est séchée par atomisation ; la poudre obtenue possède un bon pouvoir détergent, et peut être utilisée en tant que shampooing sec pour le nettoyage des cheveux et du cuir chevelu.

### Exemple 8 :

| | |
|---|---|
| Huile minérale | 4,50 % |
| Conservateur | 0,5 % |
| Caséinate de sodium | 5,4 % |
| Eau | qsp 100 % |
| Gomme de xanthane et de caséine | 3,1 % |
| Lauryl éthoxy sulfate de sodium | 8,9 % |

Après homogénéisation, l'émulsion H/E est séchée par atomisation. La poudre obtenue est bien pulvérulente et non grasse, elle permet d'obtenir une poudre de nettoyage moussante et rinçable à l'eau.

### Exemple 9 :

| | |
|---|---|
| Mélange huile de tournesol, hybride de tournesol, rosier muscat, pépins de cassis (31/60/5,95/3) | 5 % |
| Fraction liquide de beurre de karité | 3,4 % |
| Esters glycériques d'acides gras essentiels (linoléique/oléique/linoléinique) | 3,5 % |
| Conservateurs | 0,6 % |
| Gomme de xanthane | 0,2 % |
| Isohexadécane | 3,5 % |
| Eau | qsp 100 % |
| Protéine hydrolysée de soja | 7,9 % |

Après homogénéisation, l'émulsion H/E est séchée par atomisation, la poudre obtenue est blanche, possède un toucher doux et une prise agréable non grasse.

A l'application cette poudre libère lentement l'huile qu'elle contient permettant de protéger et nourrir la peau. Cette poudre peut être utilisée telle que en tant que poudre de soin.

### Exemple 10:

| | |
|---|---|
| Beurre de karité | 1,5 % |
| Octanoate cétéarylique et myristate d'isopropyle | 5 % |
| Conservateurs | 0,6 % |
| Gomme de xanthane | 0,2 % |
| Diméthicone | 3,5 % |
| Eau | qsp 100 % |
| Acide ascorbique | 5 % |
| Protéine de soja hydrolysée | 2,8 % |

Après homogénéisation de l'émulsion, on sèche l'émulsion par atomisation, on obtient une poudre blanche non grasse et non collante qui permet d'avoir une bonne stabilité de la vitamine C pendant le stockage.

La poudre obtenue peut être utilisée en tant que telle ou reconstituée pour obtenir un produit efficace pour la fermeté de la peau, et la dépigmentation des taches pigmentaires dues au soleil ou au vieillissement.

### Exemple 11:

| | |
|---|---|
| Huile d'abricot | 1 % |
| Beurre de shoréa | 5 % |
| Octanoate cétéarylique et myristate d'isopropyle | 5 % |
| Rétinol | 0,5 % |
| Conservateurs | 0,6 % |
| Gomme de xanthane | 0,2 % |
| Diméthicone | 3,5 % |
| Eau | qsp 100 % |
| Protéine de soja et gomme de xanthane | 3,5 % |
| Protéine de soja hydrolysée | 3,6 % |

Après homogénéisation, l'émulsion est séchée par atomisation, on obtient une poudre blanche non collante et non grasse à la prise du produit, qui permet d'avoir une bonne stabilité du rétinol pendant le stockage du produit.

La poudre peut être utilisée en tant que telle, non reconstituée pour obtenir un produit efficace contre les signes du vieillissement.

## Revendications

1. Utilisation d'un agent servant à la fois d'agent structurant et émulsionnant choisi parmi les biopolymères, dans une poudre cosmétique ou dermatologique contenant outre cet agent, au moins une matière grasse, au moins une substance cosmétiquement ou dermatologiquement active, la dite poudre contenant au moins 1% en poids de matière grasse et de substances actives, la dite poudre étant susceptible d'être obtenue à partir d'une émulsion huile-dans-eau homogénéisée et déshydratée, et ladite poudre étant susceptible de reconstituer par hydratation ladite émulsion huile-dans-eau.

2. Utilisation selon la revendication 1, caractérisée en ce que l'humidité résiduelle de la poudre se situe entre 2 et 4%.

3. Utilisation selon l'une des revendications 1 ou 2, caractérisée en ce que la poudre contient entre 1 et 94 % de matière grasse et de substances cosmétiquement actives.

4. Utilisation selon la revendication 3, caractérisée en ce que la poudre contient entre 10 et 70 % de matière grasse et de substances cosmétiquement actives.

5. Utilisation selon l'une des revendications 1 à 4, caractérisée en ce que la poudre contient entre 4 et 30 % d'agent structurant et émulsionnant.

6. Utilisation selon la revendication 5, caractérisée en ce que la poudre contient entre 4 et 15 % d'agent structurant et émulsionnant.

7. Utilisation selon l'une des revendications 1 à 6, caractérisée en ce que la matière grasse est choisie parmi l'huile de coco, d'amandes douces, d'abricot, de maïs, de jojoba, d'olive, d'avocat, de suif, de sésame, de palme, de paraffine, de vaseline, de silicone, les cires, les esters d'acide gras et les esters d'alcool gras.

8. Utilisation selon l'une des revendications précédentes, caractérisée en ce que la poudre contient, en outre un agent choisi parmi les hydrocolloïdes.

9. Utilisation selon l'une des revendications précédentes, caractérisée en ce que la substance cosmétiquement ou dermatologiquement active est choisie parmi les agents anti-acné, anti-microbiens, anti-transpiration, d'astringence, déodorants, dépilatoire, analgésique externe, les agents de conditionnement des cheveux, de conditionnement de la peau, de protection solaire, les vitamines, les catéchines, les flavonoïdes, les céramides, les matières grasses, les acides gras polyinsaturés, les acides gras essentiels, les agents kératolytiques, les enzymes, les anti-enzymes, les hydratants, les anti-inflammatoires, les détergents, les parfums, les charges matifiantes minérales ou synthétiques.

10. Utilisation selon l'une des revendications précédente, caractérisée en ce que la poudre contient de 0 à 30% d'un agent de surface.

11. Poudre cosmétique ou dermatologique obtenue à partir d'une émulsion huile-dans-eau homogénéisée et déshydratée, la dite poudre contenant au moins un biopolymère servant à la fois d'agent structurant et d'agent émulsionnant, choisi parmi les protéines d'origine animale ou végétale et leurs dérivés ou hydrolysats, au moins un polysaccharide à l'exception des cyclodextrines, servant à la fois d'agent structurant et d'agent émulsionnant, au moins une matière grasse, au moins une substance cosmétiquement ou dermatologiquement active, la dite poudre contenant entre 10 et 70 % de matière grasse et de substances cosmétiquement actives, entre 4 et 30 % d'agent structurant et émulsionnant et étant susceptible de reconstituer par hydratation ladite émulsion huile-dans-eau.

12. Poudre selon la revendication 11, caractérisée en ce que la matière grasse est choisie parmi l'huile de coco, d'amandes douces, d'abricot, de maïs, de jojoba, d'olive, d'avocat, de suif, de sésame, de palme, de paraffine, de vaseline, de silicone, les cires, les esters d'acide gras et les esters d'alcool gras.

13. Poudre selon la revendication 11 ou 12, caractérisée en ce que la substance cosmétiquement ou dermatologiquement active est choisie parmi les agents anti-acné, anti-microbiens, anti-transpiration, d'astringence, déodorants, dépilatoire, analgésique externe, les agents de conditionnement des cheveux, de conditionnement de la peau, de protection solaire, les vitamines, les catéchines, les flavonoïdes, les céramides, les matières grasses, les acides gras polyinsaturés, les acides gras essentiels, les agents kératolytiques, les enzymes, les anti-enzymes, les hydratants, les anti-inflammatoires, les détergents, les parfums, les charges matifiantes minérales ou synthétiques.

14. Poudre selon l'une quelconque des revendications 11 à 13, caractérisée en ce qu'elle contient de 0 à 30% d'un agent de surface.

15. Poudre selon l'une quelconque des revendications 11 à 14, caractérisée en ce qu'elle est encapsulée dans des capsules ingérables.

16. Utilisation d'une poudre ayant une humidité résiduelle de 2 à 4 % et contenant entre 4 et 30 % d'un biopolymère servant à la fois d'agent structurant et émulsionnant choisi parmi les protéines d'origine animale ou végétale et leurs dérivés ou hydrolysats, au moins une matière grasse et au moins une substance cosmétiquement ou dermatologiquement active, la ou les matières grasses et substances actives étant présentes en une quantité comprise entre 10 et 70 % du poids total de la poudre, pour reconstituer une émulsion huile-dans-eau stable par addition de liquide dans un rapport poudre/liquide compris entre 10:1 et 1:1.

## Claims

1. Use of an agent which acts both as a structuring agent and an emulsifier, chosen from biopolymers, in a cosmetic or dermatological powder containing, besides this agent, at least one fatty substance, at least one cosmetically or dermatologically active substance, the said powder containing at least 1% by weight of fatty substance and of active substances, it being possible for the said powder to be obtained from a homogenized and dehydrated oil-in-water emulsion, and it being possible for the said powder to reconstitute the said oil-in-water emulsion by hydration.

2. Use according to Claim 1, characterized in that the residual moisture content of the powder is between 2 and 4%.

3. Use according to either of Claims 1 and 2, characterized in that the powder contains between 1 and 94% of fatty substance and of cosmetically active substances.

4. Use according to Claim 3, characterized in that the powder contains between 10 and 70% of fatty substance and of cosmetically active substances.

5. Use according to one of Claims 1 to 4, characterized in that the powder contains between 4 and 30% of emulsifying and structuring agent.

6. Use according to Claim 5, characterized in that the powder contains between 4 and 15% of emulsifying and structuring agent.

7. Use according to one of Claims 1 to 6, characterized in that the fatty substance is chosen from coconut oil, sweet almond oil, apricot oil, corn oil, jojoba oil, olive oil, avocado oil, tallow oil, sesame oil, palm oil, liquid paraffin, liquid petrolatum, silicone oil, waxes, fatty acid esters and fatty alcohol esters.

8. Use according to one of the preceding claims, characterized in that the powder also contains an agent chosen from hydrocolloids.

9. Use according to one of the preceding claims, characterized in that the cosmetically or dermatologically active substance is chosen from anti-acne agents, antimicrobial agents, antiperspiration agents, astringents, deodorants, hair removers, external analgesics, hair conditioners, skin conditioners, antisun agents, vitamins, catechins, flavonoids, ceramides, fatty substances, polyunsaturated fatty acids, essential fatty acids, keratolytic agents, enzymes, anti-enzymes, moisturizers, anti-inflammatory agents, detergents, fragrances and inorganic or synthetic matt-effect fillers.

10. Use according to one of the preceding claims, characterized in that the powder contains from 0 to 30% of a surface agent.

11. Cosmetic or dermatological powder obtained from a homogenized and dehydrated oil-in-water emulsion, the said powder containing at least one biopolymer acting both as a structuring agent and as an emulsifier, chosen from proteins of animal or plant origin and derivatives or hydrolysates thereof, at least one polysaccharide with the exception of cyclodextrins, acting both as a structuring agent and an emulsifier, at least one fatty substance, at least one cosmetically or dermatologically active substance, the said powder containing between 10 and 70% of fatty substance and of cosmetically active substances, between 4 and 30% of structuring agent and emulsifier and being capable of reconstituting the said oil-in-water emulsion by hydration.

12. Powder according to Claim 11, characterized in that the fatty substance is chosen from coconut oil, sweet almond oil, apricot oil, corn oil, jojoba oil, olive oil, avocado oil, tallow oil, sesame oil, palm oil, liquid paraffin, liquid petrolatum, silicone oil, waxes, fatty acid esters and fatty alcohol esters.

13. Powder according to Claim 11 or 12, characterized in that the cosmetically or dermatologically active substance is chosen from anti-acne agents, antimicrobial agents, antiperspiration agents, astringents, deodorants, hair removers, external analgesics, hair conditioners, skin conditioners, antisun agents, vitamins, catechins, flavonoids, ceramides, fatty substances, polyunsaturated fatty acids, essential fatty acids, keratolytic agents, enzymes, anti-enzymes, moisturizers, anti-inflammatory agents, detergents, fragrances and inorganic or synthetic matt-effect fillers.

14. Powder according to any one of Claims 11 to 13, characterized in that it contains from 0 to 30% of a surface agent.

15. Powder according to any one of Claims 11 to 14, characterized in that it is encapsulated in ingestible capsules.

16. Use of a powder having a residual moisture content of 2 to 4% and containing between 4 and 30% of a biopolymer which acts both as a structuring agent and an emulsifier, chosen from proteins of animal or plant origin and derivatives or hydrolysates thereof, at least one fatty substance and at least one cosmetically or dermatologically active substance, the fatty substance or substances and active substance or substances being present in an amount of between 10 and 70% of the total weight of the powder, in order to reconstitute a stable oil-in-water emulsion by addition of liquid in a powder/liquid ratio of between 10:1 and 1:1.

## Patentansprüche

1. Verwendung eines gleichzeitig strukturierend und emulgierend wirkenden Mittels, das unter Biopolymeren ausgewählt ist, in einem kosmetischen oder dermatologischen Pulver, das neben diesem Mittel mindestens eine Fettsubstanz und mindestens einen kosmetischen oder dermatologischen Wirkstoff enthält, wobei das Pulver mindestens 1 Gew.-% Fettsubstanz und Wirkstoffe enthält, ausgehend von einer homogenisierten und dehydratisierten Öl-in-Wasser-Emulsion erhältlich ist und durch Hydratation zu einer Öl-in-Wasser-Emulsion rekonstituierbar ist.

2. Verwendung nach Anspruch 1, dadurch gekennzeichnet, daß die Restfeuchtigkeit des Pulvers 2 bis 4% beträgt.

3. Verwendung nach einem der Ansprüche 1 oder 2, dadurch gekennzeichnet, daß das Pulver 1 bis 94% Fettsubstanzen und kosmetische Wirkstoffe enthält.

4. Verwendung nach Anspruch 3, dadurch gekennzeichnet, daß das Pulver 10 bis 70% Fettsubstanzen und kosmetische Wirkstoffe enthält.

5. Verwendung nach einem der Ansprüche 1 bis 4, dadurch gekennzeichnet, daß das Pulver 4 bis 30 Gew.-% strukturierende und emulgierende Wirkstoffe enthält.

6. Verwendung nach Anspruch 5, dadurch gekennzeichnet, daß das Pulver 4 bis 15 Gew.-% strukturierende und emulgierende Mittel enthält.

7. Verwendung nach einem der Ansprüche 1 bis 6, dadurch gekennzeichnet, daß die Fettsubstanz unter Kokosöl, Süßmandelöl, Aprikosenöl, Maisöl, Jojobaöl, Olivenöl, Avocadoöl, Talgöl, Sesamöl, Palmöl, Paraffinöl, Vaselineöl, Siliconöl, Wachsen, Fettsäureestern und Estern von Fettalkoholen ausgewählt ist.

8. Verwendung nach einem der vorstehenden Ansprüche, dadurch gekennzeichnet, daß das Pulver ferner ein unter Hydrokolloiden ausgewähltes Mittel enthält.

9. Verwendung nach einem der vorstehenden Ansprüche, dadurch gekennzeichnet, daß der kosmetische oder dermatologische Wirkstoff unter Antiaknemitteln, antimikrobiellen Mitteln, Antitranspirationsmitteln, adstringierenden Mitteln, desodorierenden Mitteln, Enthaarungsmitteln, äußerlich wirkenden Analgetika, Konditionierungsmitteln für Haare, Konditionierungsmitteln für die Haut, Sonnenschutzmitteln, Vitaminen, Katechinen, Flavonoiden, Ceramiden, Fettsubstanzen, mehrfach ungesättigten Fettsäuren, essentiellen Fettsäuren, keratolytischen Mitteln, Enzymen, Enzymhemmstoffen, hydratisierenden Mitteln, entzündungshemmenden Mitteln, Detergentien, Parfums und anorganischen oder synthetischen mattierenden Füllstoffen ausgewählt ist.

10. Verwendung nach einem der vorstehenden Ansprüche, dadurch gekennzeichnet, daß das Pulver 0 bis 30% eines oberflächenaktiven Mittels enthält.

11. Kosmetisches oder dermatologisches Pulver, erhalten aus einer homogenisierten und dehydratisierten Öl-in-Wasser-Emulsion, wobei das Pulver mindestens ein Biopolymeres, das gleichzeitig als strukturierendes und emulgierendes Mittel wirkt und unter Proteinen tierischen oder pflanzlichen Ursprungs und deren Derivaten oder Hydrolysaten ausgewählt ist, mindestens ein Polysaccharid mit Ausnahme von Cyclodextrinen, das gleichzeitig als strukturierendes und emulgierendes Mittel wirkt, mindestens eine Fettsubstanz und mindestens einen kosmetischen oder dermatologischen Wirkstoff enthält, wobei das Pulver 10 bis 70% Fettsubstanz und kosmetische Wirkstoffe und 4 bis 30% strukturierendes und emulgierendes Mittel enthält und durch Hydratation zu einer Öl-in-Wasser-Emulsion rekonstituiert werden kann.

12. Pulver nach Anspruch 11, dadurch gekennzeichnet, daß die Fettsubstanz unter Kokosöl, Süßmandelöl, Aprikosenöl, Maisöl, Jojobaöl, Olivenöl, Avocadoöl, Talgöl, Sesamöl, Palmöl, Paraffinöl, Vaselineöl, Siliconöl, Wachsen, Fettsäureestern und Estern von Fettalkoholen ausgewählt ist.

13. Pulver nach Anspruch 11 oder 12, dadurch gekennzeichnet, daß der kosmetische oder dermatologische Wirkstoff unter Antiaknemitteln, antimikrobiellen Mitteln, Antitranspirationsmitteln, adstringierenden Mitteln, desodorierenden Mitteln, Enthaarungsmitteln, äußerlich anzuwendenden Analgetika, Konditionierungsmitteln für Haare, Konditionierungsmitteln für die Haut, Sonnenschutzmitteln, Vitaminen, Katechinen, Flavonolden, Ceramiden, Fettsubstanzen, mehrfach ungesättigten Fettsäuren, essentiellen Fettsäuren, keratolytischen Mitteln, Enzymen, Enzymhemmstoffen, hydratisierenden Mitteln, entzündungshemmenden Mitteln, Detergentien, Mitteln, Parfums, anorganischen oder synthetischen mattierenden Füllstoffen ausgewählt wird .

14. Pulver nach einem der Ansprüche 11 bis 13, dadurch gekennzeichnet, daß es 0 bis 30% eines oberflächenaktiven Mittels enthält.

15. Pulver nach einem der Ansprüche 11 bis 14, dadurch gekennzeichnet, daß es in zum Einnehmen bestimmten Kapseln eingekapselt ist.

16. Verwendung eines Pulvers mit einer Restfeuchtigkeit von 2 bis 4%, das 4 bis 30% eines Biopolymeren, das gleichzeitig als strukturierendes und emulgierendes Mittel wirkt und unter Proteinen tierischen oder pflanzlichen Ursprungs und deren Derivaten und Hydrolysaten ausgewählt ist, mindestens eine Fettsubstanz und mindestens einen kosmetischen oder dermatologischen Wirkstoff enthält, wobei die Fettsubstanz(en) und die Wirkstoffe in einer Menge von 10 bis 70 Gew.-%, bezogen auf das Gesamtgewicht des Pulvers, enthalten sind, zur Rekonstitution einer stabilen Öl-in-Wasser-Emulsion durch Zugabe der Flüssigkeit in einem Verhältnis von Pulver/Flüssigkeit von 10:1 bis 1:1.
